# EUROPEAN PATENT SPECIFICATION

(11) **EP 2 149 564 B1**
(45) Date of publication and mention of the grant of the patent: **25.12.2013**
(21) Application number: 08748648.6
(22) Date of filing: 22.05.2008
(51) Int. Cl.: C07D 239/47, A01N 43/54, A01N 43/56, A01N 43/78, A01N 47/24

(54) **SUBSTITUTED PYRIMIDINE ETHER COMPOUNDS AND THEIR USE**
SUBSTITUIERTE PYRIMIDINETHERVERBINDUNGEN UND IHRE VERWENDUNG
COMPOSÉS D'ÉTHER DE PYRIMIDINE SUBSTITUÉ ET LEUR UTILISATION

(30) Priority: 25.05.2007 CN 200710011434
(43) Date of publication of application: 03.02.2010
(73) Proprietor: Sinochem Corporation, Beijing 100045 (CN); Shenyang Research Institute of Chemical Industry Co., Ltd., Shenyang Liaoning 110021 (CN)
(72) Inventor: LIU, Changling, Liaoning 110021 (CN); CHAI, Baoshan, Liaoning 110021 (CN); ZHANG, Hong, Liaoning 110021 (CN); LI, HuiChao, Liaoning 110021 (CN); WANG, Junfeng, Liaoning 110021 (CN); PENG, Yongwu, Liaoning 110021 (CN); YANG, Jichun, Liaoning 110021 (CN); LIU, Guangxin, Liaoning 110021 (CN); LI, Zhinian, Liaoning 110021 (CN)
(74) Representative: Stuart, Ian Alexander
(86) International application number: PCT/CN2008/071042
(87) International publication number: WO 2008/145052

(56) References cited:
- EP-A1- 1 070 692
- EP-A2- 0 264 348
- EP-A2- 0 431 328
- EP-A2- 0 513 580
- WO-A1-2005/123054
- DE-A1- 4 029 650
- JP-A- 62 099 305
- US-A- 5 106 852
- US-A- 5 298 527
- US-A- 5 935 965
- US-A- 6 114 342
- H. SAUTER ET AL.: "Strobilurins: Evolution of a New Class Of Active Substances", ANGEWANDTE CHEMIE INTERNATIONAL EDITION, vol. 38, no. 10, 11 May 1999 (1999-05-11), pages 1328-1349, XP002662235,
- B.-S- CHAI ET AL.: "Design, synthesis and acaricidal activity of novel strobilurin derivatives containing pyrimidine moieties", PEST MANAG. SCI., vol. 66, no. 11, 2 August 2010 (2010-08-02), pages 1208-1214, DOI: 10.1002/ps.1997

## Description

### FIELD OF THE INVENTION

The invention relates to substituted pyrimidine ether compounds and use thereof, particularly as insecticide, acaricide or fungicide.

### BACKGROUND OF THE INVENTION

Methoxyacrylate compounds are natural products and known with biological activity. Methoxyacrylate compounds with insecticidal and acaricidal activities were reported as follows: EP242081, EP299694, EP335519, US2006235075, etc. In addition, strobilurins containing pyrimidine moiety also show insecticidal, acaricidal and fungicidal activity:

The following compounds with insecticidal activity were known in patent US5106852:

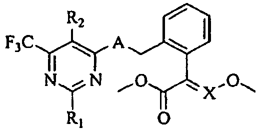

Wherein: R₁ is selected from alkyl, cycloalkyl, haloalkyl, alkoxy, alkylthio, substituted or unsubstituted aryl.

The following compound with fungicidal activity was disclosed in US5378711:

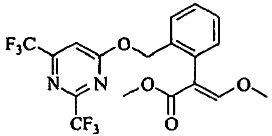

The following compounds with acaricidal, fungicidal activity were known in patent US5935965:

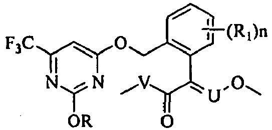

The following compounds with insecticidal, fungicidal activity were known in patent US6114342:

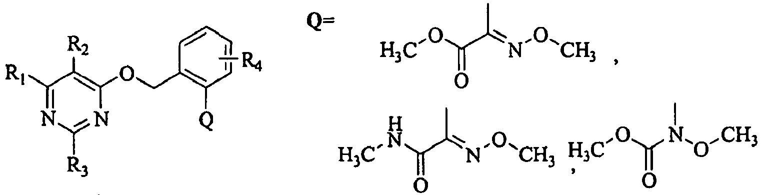

US-A-5 298 527 discloses compounds with insecticidal/fungicidal properties including compounds of the form:

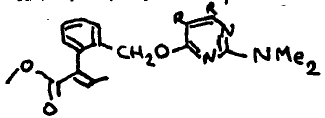

The compounds disclosed above were similar to this invention, but there are some obvious differences in structures.

### SUMMARY OF THE INVENTION

The aim of the present invention is to provide the substituted pyrimidine ether compounds with biological activity against all sorts of plant disease and insects at very low dosage and the compounds can be applied in agriculture to control disease and insects in plant.

Detailed description of the invention is as follows:

The present invention offers substituted pyrimidine ether compounds having general formula **I** as set out in claim 1.

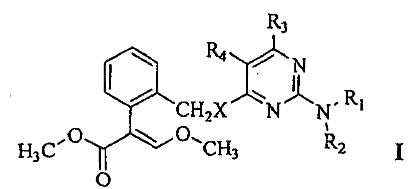

The following is the meaning of terms in the general formula I:
Halogen or halo is fluorine, chlorine, bromine or iodine.

The alkyl is to be understood as meaning straightchain or branched alkyl, such as methyl, ethyl, propyl, isopropyl or tert-butyl.

The cycloalkyl is unsubstituted or optionally substituted cyclic alkyl, such as cyclopropyl, cyclopentyl or cyclohexyl. The substituted group is methyl or halogen.

The haloalkyl refers to straight or branched chain alkyl, in which hydrogen atoms may be all or partly substituted with halogen, such as chloromethyl, dichloromethyl, trichloromethyl, fluoromethyl, difluoromethyl, trifluoromethyl.

The alkoxy refers to straight or branched chain alkyl, which is linked to the structure by oxygen atom.

The haloalkoxy refers to straight or branched chain alkoxy, in which hydrogen atoms may be all or partly substituted with halogen, such as chloromethoxy, dichloromethoxy, trichloromethoxy, fluoromethoxy, difluoromethoxy, trifluoromethoxy, chlorofluoromethoxy or trifluoroethoxy.

Because of the C=C link to -OCH₃, the compounds of the invention may form geometrical isomers (the different isomers are respectively expressed with Z and E). Z isomer and E isomer and their mixtures in any proportion are included in the invention.

When R₂ is pyridinyl, it may be substituted with groups shown in Tables 1-3. When R₂ is phenyl, it may be substituted with groups shown in Table 4. When R₂ is benzyl, it may be substituted with groups shown in Table 5. When NR₁R₂ join together to form a ring, the compounds may include groups as shown in Table 6. The groups in general formula I R₁, R₃, R₄ and X, are defined as above.

Q represents 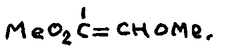 .

**TABLE 1**

| | | | | |
|---|---|---|---|---|
| 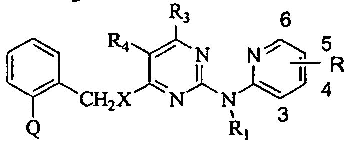 | | | | |

| R | R | R | R | R |
|---|---|---|---|---|
| H | 3-Br | 6-OCH₃ | 3-CH₃-5-NO₂ | 6-CH₃-3,5-2Br |
| 3-CH₃ | 4-Br | 5-OCH₃ | 4-CH₃-3-NO₂ | |
| 4-CH₃ | 5-Br | 3,5-2Cl | 4-CH₃-5-NO₂ | 4-CH₃-5-NO₂-3-Br |
| 5-CH₃ | 6-Br | 3,5-2Br | 5-CH₃-3-NO₂ | 3-CN-4,6-2Cl |
| 6-CH₃ | 5-I | 4-CH₃-5-Br | 6-CH₃-4-NO₂ | 3-CN-4-CH₃-6-Cl |
| 3-Cl | 5-F | 6-CH₃-5-CN | 6-CH₃-5-NO₂ | 3-CN-4-CF₃-6-Cl |
| 4-Cl | 6-F | 3,5,6-3Cl | 3-NO₂-5-Cl | 4-CH₃-5-CN-6-Cl |
| 5-Cl | 3-CN | | 3-NO₂-5-Br | 4-CF₃-5-CN-6-Cl |
| 6-Cl | 4-CN | | 5-NO₂-3-Br | |
| 3-CF₃ | 5-CN | | 5-CH₃-3-Br | |
| 4-CF₃ | 6-CN | 5-CF₃-3-Cl | 6-CH₃-5-Br | 5-CF₃-3,6-2Cl |
| 5-CF₃ | 3-NO₂ | 5-CN-3-Cl | 3-CH₃-5-Br | 5-CF₃-6-Cl |
| 6-CF₃ | 5-NO₂ | 5-CH₃-3-Cl | 3-CF₃-6-Cl | 3-CN-6-Cl |

**TABLE 2**

| | | | | |
|---|---|---|---|---|
| 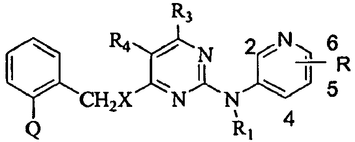 | | | | |

| R | R | R | R | R |
|---|---|---|---|---|
| H | 2-OCH₃ | 4-CH₃-2-Cl | 4-CF₃-2,6-2C1 | |
| 2-Cl | 6-OCH₃ | 5-CH₃-2-Cl | 4-CH₃-2,6-2Cl | 6-OCH₃-2-Cl |
| 6-Cl | | 6-CH₃-2-Cl | | |
| 2-Br | 2,6-2Cl | | 2-OCH₂CF₃ | |
| 6-Br | 5,6-2Cl | | 4-CH₃ | |
| | 2,5-2Cl | 4-CF₃ | 6-CF₃ | |

**TABLE 3**

| | | | | |
|---|---|---|---|---|
| 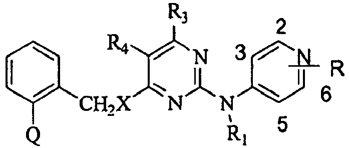 | | | | |

| R | R | R | R | R |
|---|---|---|---|---|
| H | 3-Cl | 2-OCH₃ | 2,6-2Cl | 2-OCH₃-6-Cl |
| 2-Cl | 2-Br | 2,6-2OCH₃ | 6-OPh | 2-NHCH₃-6-Cl |

**TABLE 4**

| | | | | | | |
|---|---|---|---|---|---|---|
| 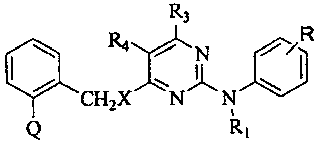 | | | | | | |

| R | R | | R | R | R | R |
|---|---|---|---|---|---|---|
| H | 2-NO₂ | | 2,3-2F | 2,3-2CH₃ | 2-Cl-4-F | 4-CH₃-2-Br |
| 2-F | 3-NO₂ | | 2,4-2F | 2,4-2CH₃ | 2-Cl-4-Br | 4-CH₃-2-Cl |
| 3-F | 4-NO₂ | | 2,5-2F | 2,5-2CH₃ | 2-Cl-4-I | 2,4,6-3CH₃ |
| 4-F | 2-SCF₃ | | 2,6-2F | 2,6-2CH₃ | 3-Cl-4-I | 2,4,6-3C₂H₅ |
| 2-Cl | 3-SCF₃ | | 3,4-2F | 3,4-2CH₃ | 4-Cl-2-Br | |
| 3-Cl | 4-SCF₃ | | 3,5-2F | 3,5-2CH₃ | 3,4,5-3F | |
| 4-Cl | 2-OC₂H₅ | | 2,3-2Cl | 2,3-2C₂H₅ | 2,3,4-3Cl | |
| 2-Br | 3-OC₂H₅ | | 2,4-2Cl | 2,4-2C₂H₅ | 2,3,5-3Cl | |
| 3-Br | 4-OC₂H₅ | | 2,5-2Cl | 2,5-2C₂H₅ | 2,3,6-3Cl | |
| 4-Br | 2-COCH₃ | | 2,6-2Cl | 2,6-2C₂H₅ | 2,4,5-3Cl | |
| 2-I | 3-COCH₃ | | 3,4-2Cl | 3,4-2C₂H₅ | 2,4,6-3Cl | |
| 3-I | 4-COCH₃ | | 3,5-2Cl | 3,5-2C₂H₅ | 3,4,5-3Cl | |
| 4-I | | 2-N(C₂H₅)₂ | 2,3-2Br | 2,3-2CF₃ | 2,3,4-3Br | |
| 2-CH₃ | | 3-N(C₂H₅)₂ | 2,4-2Br | 2,4-2CF₃ | 2,3,5-3Br | 2-CH(CH₃)₂ |
| 3-CH₃ | | 4-N(C₂H₅)₂ | 2,5-2Br | 2,5-2CF₃ | 2,3,6-3Br | 3-CH(CH₃)₂ |
| 4-CH₃ | | 4-Ph | 2,6-2Br | 2,6-2CF₃ | 2,4,5-3Br | 4-CH(CH₃)₂ |
| 2-C₂H₅ | | 2-OPh | 3,4-2Br | 3,4-2CF₃ | 2,4,6-3Br | 2-CF₃-4-Cl |
| 3-C₂H₅ | | 3-OPh | 3,5-2Br | 3,5-2CF₃ | 3,4,5-3Br | 2-CF₃-4-Br |
| 4-C₂H₅ | | 4-OPh | 2,3-2CN | | 4-CH₃-3-F | 3-CF₃-4-NO₂ |
| 2-CF₃ | | 2,3-2OCH₃ | 2,4-2CN | | 4-CH₃-3-Cl | 3-CF₃-4-F |
| 3-CF₃ | | 2,4-2OCH₃ | 2,5-2CN | | 4-CH₃-3-Br | 3-CF₃-4-Cl |
| 4-CF₃ | | 2,5-2OCH₃ | 2,6-2CN | | 2,4,6-3CF₃ | 4-CF₃-2-NO₂ |
| 2-OCH₃ | | 2,6-2OCH₃ | 3,4-2CN | | 2-CH₃-3-F | 4-CF₃-2-Cl |
| 3-OCH₃ | | 3,4-2OCH₃ | 3,5-2CN | | 2-CH₃-3-Cl | 4-CF₃-2-Br |
| 4-OCH₃ | | 3,5-2OCH₃ | 2-F-4-CI | | 2-CH₃-4-F | 2-CH₃-5-NO₂ |
| | | | 2-F-4-Br | | 2-CH₃-4-Cl | 2-CH₃-3-NO₂ |
| | | | 2-F-4-I | | 2-CH₃-4-Br | |
| | | | 2-F-5-Cl | 2,3-2OCF₃ | 2-CH₃-5-F | |
| 2-OCF₃ | | | 3-F-5-Cl | 2,4-2OCF₃ | 2-CH₃-5-Cl | 2-CH₃-4-NO₂ |
| 3-OCF₃ | | | 4-F-3-Cl | 2,5-2OCF₃ | 2-CH₃-5-Br | 2-CH₃-4-OCH₃ |
| 4-OCF₃ | | 2,3-2NO₂ | 4-F-6-Cl | 2,6-2OCF₃ | 2-CH₃-6-Cl | 2-CH₃-6-C₂H₅ |
| 2-CN | | 2,4-2NO₂ | 2,3,4-3F | 3,4-2OCF₃ | 3-CH₃-2-Br | 2-CH₃-6-NO₂ |
| 3-CN | | 2,5-2NO₂ | 2,3,5-3F | 3,5-2OCF₃ | 3-CH₃-4-Cl | 2,4,6-3NO₂ |
| 4-CN | 2-OCHF₂ | 2,6-2NO₂ | 2,3,6-3F | | 3-CH₃-4-Br | 2,3-2Cl-4-Br |
| | 3-OCHF₂ | 3,4-2NO₂ | 2,4,5-3F | | 3-CH₃-4-I | 2,4-2F-6-Cl |
| | 4-OCHF₂ | 3,5-2NO₂ | 2,4,6-3F | | 2-CH₃-4-I | 2-NO₂-4,6-2Br |

| R | R | R | R | R | | |
|---|---|---|---|---|---|---|
| 5-CF₃-2-Cl | 5-CF₃-2-OCH₃ | 4-CH₃-2,6-2Br | | 2-NO₂-4-F | | |
| 5-CF₃-2-Br | 2-CF₃-4-NO₂ | 5-CH₃-4-F-6-Cl | | 2-NO₂-4-Cl | | |
| 2-CN-3-F | 2,4-2NO₂-6-Cl | 4-C(CH₃)₃-2-Cl | | 2-NO₂-4-Br | | |
| 2-CN-3-Cl | 2,4-2NO₂-6-Br | 4-CF₃-2-Cl-6-Br | 5-CH₃-2-OCH₃-4-Cl | 2-NO₂-5-Cl | | |
| 2-CN-4-NO₂ | 2,3-2CH(CH₃)₂ | | | 3-NO₂-4-Cl | | |
| 2-CN-4-Cl | 2,4-2CH(CH₃)₂ | | | 3-NO₂-4-Br | | |
| 2-CN-4-Br | 2,5-2CH(CH₃)₂ | | 2-CH₃-4-NO₂-6-Cl | 4-NO₂-2-Cl | | |
| 4-CN-2-CF₃ | 2,6-2CH(CH₃)₂ | 2,4,6-3CH(CH₃)₂ | 2-CH₃-4-NO₂-6-Br | 5-NO₂-2-Cl | | |
| 4-CN-2-Cl | 3,4-2CH(CH₃)₂ | 2,4,6-3C(CH₃)₃ | 2-CH₃-6-NO₂-4-Cl | 5-NO₂-2-Br | | |
| 4-CN-2-NO₂ | 3,5-2CH(CH₃)₂ | 2,3-2CH₃-6-NO₂ | 2-CH₃-6-NO₂-4-Br | 2-OCH₃-5-Cl | | |
| 5-CH₃-2-F | 2-NO₂-4-OCH₃ | 2,4-2OCH₃-5-Cl | 2,5-2OCH₃-4-NO₂ | 4-OCH₃-3-F | | |
| 4-CH₃-2-NO₂ | 2-NO₂-4-OC₂H₅ | | 2,6-2CH₃-4-C(CH₃)₃ | 4-OCH₃-3-Cl | | |
| 4-CH₃-3-NO₂ | 2,3-2C(CH₃)₃ | | 4-CF₃-2-NO₂-5-Cl | 3-NO₂-4-F | | |
| 5-CH₃-2-CN | 2,4-2C(CH₃)₃ | | 4-CF₃-2-NO₂-6-Cl | 2-OCF₃-4-CN | | |
| 5-NO₂-2-F | 2,5-2C(CH₃)₃ | 4,5-2CH₃-2-NO₂ | 4-CF₃-2-NO₂-6-Br | 2-OCF₃-4-Cl | | |
| 2-CF₃-4,6-2Cl | 2,6-2C(CH₃)₃ | 2-NO₂-4-F-5-Cl | | 2-OCF₃-4-Br | | |
| 2-CF₃-4,6-2Br | 3,4-2C(CH₃)₃ | 2-CN-4-NO₂-6-Cl | | 2-F-4,6-2Br | | |
| 3-CH₃-2,6-2Cl | 3,5-2C(CH₃)₃ | 2-CN-4-NO₂-6-Br | | 4-OCF₃-2-Cl | | |
| 2-CH₃-4,6-2Br | 4-SO₂NH₂ | | | 4-OCF₃-2-Br | | |
| 2,4,6-3OCH₃ | 4-NO₂-2-OCH₃ | | | 2,3,5,6-4F | | |
| 3,4,5-3OCH₃ | | | | 2-CN-4,6-2Cl | | |
| | | | | 2-CN-4,6-2Br | | |
| 2,4,6-3OCF₃ | | | | 4-CN-2,6-2Cl | | |
| | | | | 4-CF₃-2,6-2Cl | | |
| | | 5-NO₂-2-OCH₃ | | 4-CF₃-2,6-2Br | | |
| | | 5-CH₃-2-OCH₃ | | 2,3,4,5,6-5Cl | | |
| | 4-F-2,6-2Br | 4-NO₂-2,6-2Cl | 2,3-(CH₂CH₂CH₂-) | 2,3-(OCF₂O-) | | |
| 2-F-3-Cl | 2,4-2F-6-Cl | 4-OCF₃-2-NO₂ | 2,3-(CH₂CH₂CH₂CH₂-) | 2,3-(OCH₂O-) | | |
| 3-CH3-2-Cl | 2-F-4-Cl-6-Br | 6-NO₂-2,3,4-3F | 4-NO₂-2,5-2Cl | 3,4-(OCH₂O-) | | |
| | 2,3,5,6-4F-4-CF₃ | 4-NO₂-2,6-2Br | 4-F-3-Cl-2,6-2Br | 3,4-(OCF₂O-) | | |

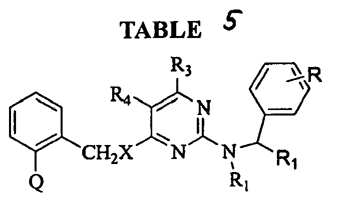

The group R in table 6 is defined as above in table 5.

**TABLE 6**

| | | |
|---|---|---|
| 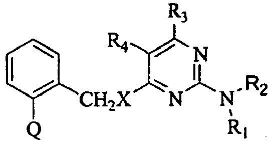 | | |

| -NR₁R₂ | -NR₁R₂ | -NR₁R₂ |
|---|---|---|
| 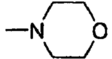 | 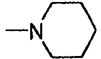 | 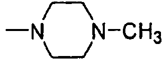 |
| 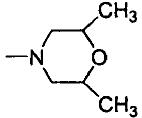 | 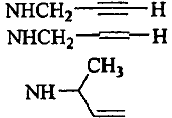 | 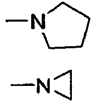 |

**TABLE 8**

| | | | | |
|---|---|---|---|---|
| 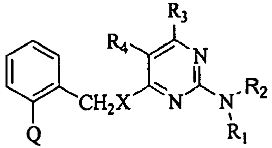 | | | | |

| R₂ | R₂ | R₂ | R₂ | R₂ |
|---|---|---|---|---|
| H | (CH₂)₆CH₃ | CF₃ | COCH₂Cl | CH₂CO₂CH₃ |
| CH₃ | (CH₂)₇CH₃ | | COCH₂Br | CH₂CO₂C₂H₅ |
| C₂H₅ | OCH₃ | | COPh | CH₂C(CH₃)₂CH₃ |
| 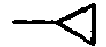 | OC₂H₅ | | COCH₃ | CH₂CH₂C(CH₃)₃ |
| (CH₂)₂CH₃ | OC(CH₃)₃ | | COC₂H₅ | CH(CH₃)CH₂CH₃ |
| CH(CH₃)₂ | OCH₂CF₃ | | CO₂CH₃ | CH₂CH(CH₃)₂ |
| (CH₂)₃CH₃ | CN | | CO₂C₂H₅ | CH₂C(CH₃)₃ |
| C(CH₃)₃ | CH₂CN | | SO₂CH₃ | NHCH₂CF₃ |
| (CH₂)₅CH₃ | CH₂CH₂CN | | SO₂C₂H₅ | NHPh-4-Cl |
| NH₂ | N(CH₃)₂ | | NHPh | CH(CH₃)CH₂CH(CH₃)₂ |
| 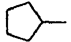 | 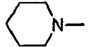 | | NHCH₂CN | CONHCOPh |
| | | | NHCOCH₂CN | CONHCOPh-2-Cl |
| 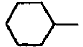 | 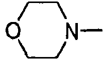 | | 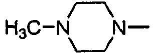 | CONHCOPh-2,6-2Cl |
| | | | | CONHCOPh-2,6-2F |

Compounds of the present invention include the following compounds in Table 7, but without being restricted thereby.

**TABLE 7**

| | | | | | |
|---|---|---|---|---|---|
| 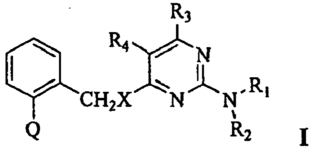 | | | | | |

| No. | X | R₁ | R₂ | R₃ | R₄ |
|---|---|---|---|---|---|
| 1 | O | H | Ph | CH₃ | H |
| 2 | O | H | Ph | CF₃ | H |
| 3 | O | H | Ph | i-Pr | H |
| 4 | O | H | Ph | t-Bu | H |
| 5 | O | H | Ph | cyclopropyl | H |
| 6 | O | H | Ph | Cl | H |
| 7 | O | H | Ph | Br | H |
| 8 | O | H | Ph | OCH₃ | H |
| 9 | O | H | Ph | OCH₂CF₃ | H |
| 10 | O | H | Ph | NO₂ | H |
| 11 | O | H | Ph | CN | H |
| 12 | O | H | Ph | CH₃ | CH₃ |
| 13 | O | H | Ph | CF₃ | CH₃ |
| 14 | O | H | Ph | i-Pr | CH₃ |
| 15 | O | H | Ph | t-Bu | CH₃ |
| 16 | O | H | Ph | cyclopropyl | CH₃ |
| 17 | O | H | Ph | CH₃ | Cl |
| 18 | O | H | Ph | CF₃ | Cl |
| 19 | O | H | Ph | i-Pr | Cl |
| 20 | O | H | Ph | t-Bu | Cl |
| 21 | O | H | Ph | cyclopropyl | Cl |
| 22 | O | H | Ph | CH₃ | Br |
| 23 | O | H | Ph | CF₃ | Br |
| 24 | O | H | Ph | i-Pr | Br |
| 25 | O | H | Ph | t-Bu | Br |
| 26 | O | H | Ph | cyclopropyl | Br |
| 27 | O | H | Ph | CH₃ | OCH₃ |
| 28 | O | H | Ph | CF₃ | OCH₃ |
| 29 | O | H | Ph | i-Pr | OCH₃ |
| 30 | O | H | Ph | t-Bu | OCH₃ |
| 31 | O | H | Ph | cyclopropyl | OCH₃ |
| 32 | 0 | H | Ph | CF₃ | NO₂ |
| 33 | O | H | Ph | CF₃ | CN |
| 34 | O | H | Ph | CF₃ | CH₂Cl |
| 35 | O | H | Ph | CF₃ | CH₂CH₂Cl |
| 36 | O | H | Ph | CF₃ | OCH₂CF₃ |
| 37 | S | H | Ph | CH₃ | H |
| 38 | S | H | Ph | CF₃ | H |
| 39 | S | H | Ph | cyclopropyl | H |
| 40 | S | H | Ph | Cl | H |
| 41 | S | H | Ph | Br | H |
| 42 | O | H | Ph | OCH₃ | H |
| 43 | S | H | Ph | OC_{H2}CF₃ | H |
| 44 | S | H | Ph | CH₃ | CH₃ |
| 45 | S | H | Ph | CF₃ | CH₃ |
| 46 | S | H | Ph | i-Pr | CH₃ |
| 47 | S | H | Ph | CH₃ | Cl |
| 48 | S | H | Ph | CF₃ | Cl |
| 49 | S | H | Ph | CF₃ | Br |
| 50 | S | H | Ph | CH₃ | OCH₃ |
| 51 | S | H | Ph | CF₃ | OCH₃ |
| 52 | S | H | Ph | CF₃ | OCH₂CF₃ |
| 53 | S | H | Ph | CF₃ | CN |
| 54 | O | H | Ph-2-Cl | CF₃ | H |
| 55 | O | H | Ph-2-CH₃ | CF₃ | H |
| 56 | O | H | Ph-2-F | CF₃ | H |
| 57 | O | H | Ph-2-C₂H₅ | CF₃ | H |
| 58 | O | H | Ph-2-CH(CH₃)₂ | CF₃ | H |
| 59 | O | H | Ph-2-OCH₃ | CF₃ | H |
| 60 | O | H | Ph-2,3-(OCH₂O-) | CF₃ | H |
| 61 | O | i-Pr | Ph | CH₃ | CH₃ |
| 62 | O | i-Pr | Ph | CF₃ | H |
| 63 | O | i-Pr | Ph | CF₃ | Cl |
| 64 | O | i-Pr | Ph | CH3 | OCH₃ |
| 65 | O | i-Pr | Ph | CF₃ | OCH₃ |
| 66 | O | H | Ph-2-Cl | CH₃ | Cl |
| 67 | O | H | Ph-2-CH₃ | CF₃ | H |
| 68 | O | H | Ph-3,5-2Cl | CF₃ | H |
| 69 | O | H | Ph-2-CH₃-3-Cl | CF₃ | H |
| 70 | O | H | Ph | CH₃ | (CH₂)₃CH₃ |
| 71 | O | H | Ph-4-CF₃ | CF₃ | H |
| 72 | O | H | Ph-2,6-2F | CF₃ | H |
| 73 | O | H | Ph-2,6-2Cl | CF₃ | H |
| 74 | O | H | Ph-3-Cl | CF₃ | H |
| 75 | O | H | Ph-4-OCH₃ | CF₃ | H |
| 76 | O | H | Ph-4-F | CF₃ | H |
| 77 | O | H | Ph-4-CH₃ | CF₃ | H |
| 78 | O | H | 2-chloro-pyridin-3-yl | CF₃ | H |
| 79 | O | H | Ph-2,4-2CH₃ | CF₃ | H |
| 80 | O | H | Ph-2,3-2Cl | CF₃ | H |
| 81 | O | H | Ph-2,5-2CH₃ | CF₃ | H |
| 82 | O | H | CH₂Ph-2-Cl | CF₃ | H |
| 83 | O | H | Ph-3,4-2CH₃ | CF₃ | H |
| 84 | O | H | Ph-2,4,5-3CH₃ | CF₃ | H |
| 85 | O | H | Ph-2-CH₃-4-Cl | CF₃ | H |
| 86 | O | H | CH₂Ph | CF₃ | H |
| 87 | O | H | Ph-4-Cl | CH₃ | H |
| 88 | O | H | Ph-4-Cl | CF₃ | H |
| 89 | O | 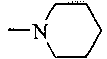 | | CF₃ | H |
| 90 | O | 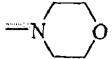 | | CF₃ | H |
| 91 | O | 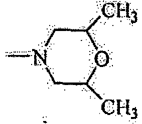 | | CF₃ | H |
| 92 | O | 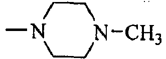 | | CF₃ | H |
| 93 | O | 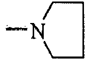 | | CF₃ | H |

The present invention also includes preparation of the compounds having formula I:

When Q is choosen from one of Q₁, Q₂, Q₄, Q₆-Q₁₃, compounds having general formula **I** and their stereoisomers can be prepared by reaction of pyrimidine compounds containing hydroxy group having general formula **III** with halomethylbenzene having general formula **IV** at the present of base:

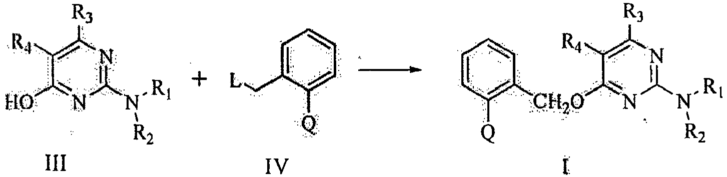

Wherein: L is leaving group, such as Cl or Br, other groups are as defined above. Intermediate of the general **IV** can be prepared according to known methods, refer to US4723034 and US5554578.

The reaction can be carried out in proper solvent, which may be selected from tetrahydrofuran, acetonitrile, toluene, xylene, benzene, DMF, DMSO, acetone or butanone and so on.

The base mentioned above may be such as potassium hydroxide, sodium hydroxide, sodium carbonate, potassium carbonate, sodium bicarbonate, triethylamine, pyridine, sodium methoxide, sodium ethoxide, sodium hydride, potassium tert-butoxide or sodium tert-butoxide.

The temperature is suitably from room temperature to boiling point of solvent, generally from 20 to 100°C.

The reaction may be finished after 30 minutes to 20 hours, 1 hour to 10 hours in general.

Suitable salts of formula **I** were obtained from the compounds of formula I with 2-aminopyrimidine group reacting with inorganic acids, for example hydrochloric or, phosphoric acid, with organic acids such as acetic acid, benzenesulfonic acid or oxalic acid.

Intermediates of the general formula **III** can be prepared by reaction of intermediates of the general formula **II** with ethyl 3-oxobutanoate or ethyl 4,4,4-trifluoro-3-oxobutanoate according to known methods, refer to GB1388402, US4000138, CH395385. Intermediates of the general formula **II** can be bought or prepared according to known methods, refer to EP310550, EP0655441.

Examples of intermediate III are listed in table 8:

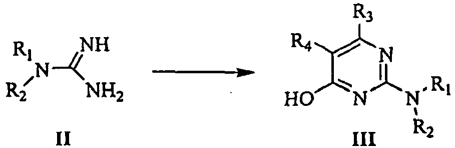

**TABLE 8 Intermediate III**

| | | | | | |
|---|---|---|---|---|---|
| 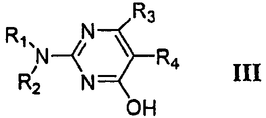 | | | | | |

| No. | R₁ | R₂ | R₃ | R₄ | m.p. (°C) |
|---|---|---|---|---|---|
| A1 | H | Ph | CH₃ | H | 258-260 |
| A2 | H | Ph | CF₃ | H | 279-281 |
| A3 | H | Ph | CH₃ | CH₃ | 282-284 |
| A4 | H | Ph | CH₃ | (CH₂)₃CH₃ | 183-185 |
| A5 | H | Ph | cyclopropyl | H | 112-115 |
| A6 | H | Ph-4-Cl | CH₃ | H | 251-253 |
| A7 | H | Ph-4-Cl | CF₃ | H | 276-278 |
| A8 | H | Ph-4-Cl | CF₃ | Cl | |
| A9 | H | Ph-4-CF₃ | CF₃ | H | 213-215 |
| A10 | H | Ph-2,6-2F | CF₃ | H | 158-160 |
| A11 | H | Ph-2,6-2Cl | CF₃ | H | 216-218 |
| A12 | H | Ph-3-Cl | CF₃ | H | 196-198 |
| A13 | H | Ph-4-OCH₃ | CF₃ | H | 156-158 |
| A14 | H | Ph-4-CH₃ | CF₃ | H | 207-209 |
| A15 | H | Ph-4-F | CF₃ | H | 204-206 |
| A16 | H | 2-chloro-pyridin-3 -yl | CF₃ | H | >295 |
| A17 | H | Ph-2-CH₃ | CF₃ | H | 222-224 |
| A18 | H | Ph-2-Cl | CF₃ | H | >290 |
| A19 | H | Ph-2-CH₃-3-Cl | CF₃ | H | 234-236 |
| A20 | H | Ph-2,4-2CH₃ | CF₃ | H | 230-232 |
| A21 | H | Ph-2,3-2Cl | CF₃ | H | 264-266 |
| A22 | H | Ph-3,5-2Cl | CF₃ | H | 287-288 |
| A23 | H | Ph-2,5-2CH₃ | CF₃ | H | 216-218 |
| A24 | H | CH₂Ph-2-Cl | CF₃ | H | 157-159 |
| A25 | H | Ph-3,4-2CH₃ | CF₃ | H | 136-137 |
| A26 | H | Ph-2,4,5-3Cl | CF₃ | H | 260-262 |
| A27 | H | Ph-2-CH₃-4-Cl | CF₃ | H | 212-214 |
| A28 | H | CH₂Ph | CF₃ | H | 152-154 |
| A29 | 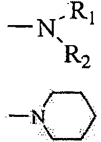 | | CF₃ | H | 168-170 |
| A30 | 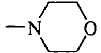 | | CF₃ | H | 261-263 |
| A31 | 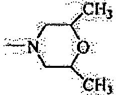 | | CF₃ | H | 220-222 |

| Intermediate No. | ¹H-NMR (300MHz, internal standard TMS, solvent CDCl₃) |
|---|---|
| A9 | δ(ppm): 5.11(s, 1H), 642(s, 1H), 7.50(m, 1H), 7.65(m, 1H), 7.76(m, 1H), 7.91(m, 1H)(solvent DMSO). |

The compounds having general formula **I** exert, with respect to the adults, larvae and eggs of mites and insects which are harmful in agricultures, a high acaricidal and insecticidal activity, while the compounds also exhibit preferably fungicidal activity.

A further object of the present invention therefore relates to the use of the compounds having general formula **I** as acaricides, insecticides, and fungicides, both in agriculture and other fields. In particular, the compounds having general formula **I** are active against important species of tetranychidae (e.g. Tetranychus urticae, Tetranychus cinnabarinus, Panonychus ulmi and Panonychus citri), eriophyidae and tarsonemidae. Additionally, some compound in present invention also exhibit good fungicidal activity, which can be used to control of fungal diseases such as rice, blast, tomato late blight, cucumber downy mildew, cucumber anthracnose, corn rust and wheat powdery mildew.

At the same time, the compounds having general formula **I** have a low toxicity with respect to many useful insects and mites, mammals, fish, birds, and have no phytotoxicity

Thanks to their positive characteristics, they can be advantageously used in defending not only crops of agrarian and horticultural interest, but also domestic and breeding animals, as well as environments frequented by human beings, from harmful mites and insects

For obtaining the desired effect, the quanlity of compounds can be varied m relation to various factors such as, the compound used, the crop to be preserved, the type of harmful organism, the degree of infestation, the climatic conditions, the method of application, the formulation adopted.

Doses of compounds ranging from 10g to 5kg per hectare generally provide a sufficient control

A further object of the present invention also relates to a method for controlling mites and/or insects and/or phytopathogenic fungi in crops of agrarian and horticultural interest and/or on domestic and breeding animals and/or environments frequented by human beings, by the application of the compounds having general formula **I**. In particular, the quantity of compounds to be applied varies from 10g to 5kg per hectare.

For practical application in agriculture, it is usually beneficail to use compositions containing one or more compounds having general formula **I**.

A further object of the present invention therefore relates to acaricidal, insecticidal and fungicidal compositions containing compounds having general formula **I** as active principle and acceptable carrier in agriculture, the active component of the compositions is 0.5-90%.

Compositions can be used in the form of dry powders, wettable powders, emulsifiable concentrates, microemulsions, pastes, granulates, solutions, suspensions, etc.: the selection of the type of compositions depends on the specific application.

The compositions are prepared in the known way, for example by diluting or dissolving the active substance with a solvent medium and/or a solid diluent, optionally in the presence of surfactant

Solid diluents, or carriers which can be used are, for example: silica, kaolin, bentonite, talc, diatomite, dolomite, calcium carbonate, magnesia, chalk, clays, synthetic silicates, attapulgite, sepiolite.

Liquid diluents which can be used are, for example, besides water, aromatic organic solvents (xylols or mixtures of alkylbenzols, chlorobenzene, etc.), paraffins (petroleum fractions), alcohols (methanol, propanol, butanol, octanol, glycerin, etc.), esters (ethyl acetate, isobutyl acetate, etc.), ketones (cyclohexanone, acetone, acetophenone, isophorone, ethylamylketone, etc.), amides (N, N-dimethylformamide, N- methylpyrrolidone, etc.).

Surfactant which can be used are salts of sodium, calcium, triethylamine or triethanolamine of alkylsulfonates, alkylarylsulfonates, polyethoxylated alkylphenols, polyethoxylated esters of sorbitol, ligninsulfonates, etc..

The compositions can also contain special additives for particular purposes, for example adhesive such as Arabic gum, polyvinyl alcohol, polyvinyl-pyrrolidone, etc..

The concentration of active principle in the above compositions can vary within a wide range depending on the active compound, the applications for which they are destined, the environmental conditions and the type of formulation adopted. In general the concentration of active principle ranges from 1 to 90%, preferably from 5 to 50%.

It is possible to add to the compositions, other active principles compatible with the compounds having general formula **I** according to requirement, such as, for example, other acaricides/insecticides, fungicides, phytoregulators, antibiotics, herbicides, fertilizers.

Several formulations of preparation methods, for example as follows:

The preparation of suspension concentrate: the common active component in formula is 5% -35%. With water as the medium, the compound, dispersing agent, suspending agent and antifreeze agent are added to sanding machine for grinding to make suspension concentrate.

The preparation of water emulsion: the compound, solvent and emulsifier are mixed together, to make a homogeneous oil phase. The water is mixed with antifreeze, as a homogeneous water phase. In the high-speed stirring, the aqueous phase added to the oil phase or oil phase is added to the aqueous phase, forming a good dispersion of the water emulsion. The active component of emulsions is generally 5% -15% in this invention. For the production of concentrated emulsions, the compounds of this invention are soluble in one or more of the mixed solvent, and then emulsifier is added to enhance dispersion effects in the water.

The preparation of wettable powder: the compound, surfactants and solid diluent are mixed well according to recipe requirements, after smashing through ultrafine pulverizer, that is the wettable powder products (for example, 10% -40%). For preparing the spraying wettable powder, the compounds of this invention can be mixed with solidpower to form the mixture, such as clay, inorganic silicates, carbonates, as well as wetting agents, adhesives and/or dispersant agent. ,

The preparation of water dispersible granules: the compound and powdered solid diluents, wetting agents and adhesives are mixed to smash, kneaded together with water and added to the granulation 10-100 mesh machine for granulation, then by drying and sieving (at the scope screen). Also, the compounds, dispersants, wetting agents, disintegrants and solid diluent are added sanding machine, grinded with water for medium to produce suspension and then spray-drying granulation, usually the preparation of content is 20% -30% granular products.

### DETAILED DESCRIPTION OF THE INVENTION

The folloing examples are illustrative of the present invention, but without being restricted thereby.

### PREPARATION EXAMPLE

### Example 1: The preparation of compound 2

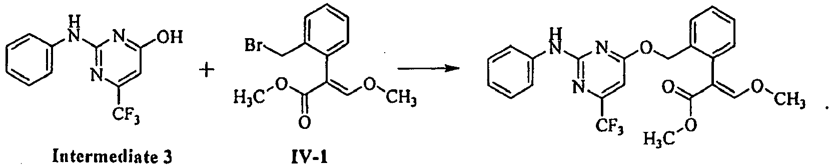

1.32 g of intermediate 3 (prepared accoriding to Bioorganic & Medicinal Chemistry Letters, 2004,14(17) : 4449-4452) was dissolved in 30 ml of butanone, 1.6 g potassium carbonate was added to the solution, which is stirred to no bubble, then 1.25 g intermediateIV-1 was added, heated to reflux, after 8 hours, the reaction was traced by Thin-Layer Chromatography, then the salt was removed by filtration, washed by ethyl acetate, the combined organic extacts were concentrated at reduced pressure. The crude product was purified through silica column (ethyl acetate/petroleum ether (boiling point range 60-90°C) = 1/5, as an eluent) and light yellow thick oil (compound 2) was obtained, which solidified to give 1.52 g product, m.p.106-109°C.

¹H-NMR spectrum(300MHz, internal standard: TMS, solvent CDCl₃) is as follows: δ(ppm): 3.70(s, 3H), 3.78(s, 3H), 5.33(s, 2H), 6.48(s, 1H), 7.07(m, 1H), 7.20(m, 1H), 7.34(m, 4H), 7.49(m, 2H), 7.58(s, 1H), 7.63(m, 2H).

Other compounds were prepared similarly.

Physical and chemical property and ¹HNMR spectrum (¹HNMR, 300MHz, internal standard:TMS, solvent CDCl₃) of some compounds having the general formula **I** of this invention are as follows:

Compound 1: thick oil. δ(ppm): 2.20(3H, s), 3.71(3H, s), 3.77(3H, s), 5.33(2H, s), 6.52(1H, s), 7.11(1H, m), 7.20(1H, m), 7.39(4H, m), 7.45(2H, m), 7.59(1H, s), 7.60(2H, m).

Compound 5: m.p. 54-56°C. δ(ppm): 1.01(m, 2H), 1.15(m, 2H), 1.83(m, 1H), 3.65(s, 3H), 3.78(s, 3H), 5.27(s, 2H), 6.06(s, 1H), 7.01(m, 1H), 7.18(m, 1H), 7.33(m, 4H), 7.51(m, 1H), 7.57(m, 1H), 7.62(m, 2H).

Compound 12: m.p. 136-138°C. δ(ppm): 2.05(s, 3H), 2.36(s, 3H), 3.68(s, 3H), 3.74(s, 3H), 5.32(s, 2H), 6.98(m, 1H), 7.20(m, 1H), 7.35(m, 4H), 7.58(m, 1H), 7.61(s, 1H), 7.63(m, 2H).

Compound 18: thick oil. δ(ppm): 3.74(s, 3H), 3.84(s, 3H), 4.49(s, 2H), 7.13(m, 1H); 7.33(m, 5H), 7.54(m, 2H), 7.60(m, 1H), 7.71(m, 1H).

Compound 44: m.p. 84-85°C. δ(ppm): 1.16(t, 6H), 3.58(q, 4H), 3.68(s, 3H), 3.80(s, 3H), 5.28(s, 2H), 6.21(s, 1H), 7.18(m, 1H), 7.34(m, 2H), 7.48(m, 1H), 7.57(s, 1H).

Compound 66: m.p. 135-137°C. δ(ppm): 3.68(s, 3H), 3.77(s, 3H), 5.38(s, 2H), 6.48(s, 1H), 6.92(m, 1H), 7.01(m, 2H), 7.20(m, 1H), 7.37(m, 2H), 7.54(m, 2H), 7.94(s, 1H).

Compound 67: m.p. 118.120°C. δ(ppm): 2.35(s, 3H), 3.66(s, 3H), 3.74(s, 3H), 5.26(s, 2H), 6.45(s, 1H), 7.08(m, 2H), 7.16(m,1H), 7.22(m, 2H), 7.34(m, 2H), 7.38(m, 1H), 7.54(s, 1H)).

Compound 68: m.p. 147-149°C. δ(ppm): 3.75(s, 3H), 3.83(s, 3H), 5.25(s, 2H), 6.55(s, 1H), 7.01(s, 1H), 7.26(m, 1H), 7.34(m, 2H), 7.44(m, 2H), 7.63(s, 1H), 7.67(s,1H).

Compound 69: m.p. 134-136°C. δ(ppm): 2.34(s, 3H), 3.68(s, 3H), 3.80(s, 3H), 5.35(s, 2H), 6.58(s, 1H), 7.22(m, 2H), 7.26(m, 1H), 7.37(m, 2H), 7.51(m, 1H), 7.58(s, 1H), 7.75(s, 1H), 8.42(d, 1H).

Compound 70: m.p. 85-88°C.δ(ppm): 0.91(t, 3H), 1.40(m, 4H), 2.40(s, 3H), 2.53(t, 2H), 3.69 s, 3H), 3.75(s, 3H), 5.24(s, 2H), 7.01(m, 1H), 7.32(m, 5H), 7.58(m, 3H), 7.61(s, 1H).

Compound 71: m.p. 149-151°C.δ(ppm): 3.74(s, 3H), 3.81(s, 3H), 5.35(s, 2H), 6.57(s, 1H), 7.21(m, 1H), 7.35(m, 2H), 7.48(m, 1H), 7.57(m, 2H), 7.62(s, 1H), 7.78(m, 2H).

Compound 72: m.p. 146-149°C.δ(ppm): 3.67(s, 3H), 3.75(s, 3H), 5.16(s, 2H), 6.48(s, 1H), 6.71(m, 1H), 7.00(m, 2H), 7.18(m, 2H), 7.32(m, 2H), 7.54(s, 1H).

Compound 73: m.p. 184-186°C.δ(ppm): 3.64(s, 3H), 3.70(s, 3H), 5.10(s, 2H), 6.46(s, 1H), 6.93(m, 1H), 7.25(m, 4H), 7.43(m, 2H), 7.53(s, 1H).

Compound 74: m.p. 125-126°C.δ(ppm): 3.73(s, 3H), 3.81(s, 3H), 5.31(s, 2H), 6.52(s, 1H), 7.00(m, 1H), 7.20 (m, 2H), 7.35(m, 2H), 7.50(m, 2H), 7.61(s, 1H), 8.79(m, 1H).

Compound 75: m.p. 137-139°C.δ(ppm): 3.70(s, 3H), 3.78(s, 3H), 3.81(s, 3H), 5.29(s, 2H), 6.43(s, 1H), 6.90(m, 2H), 7.20(m, 2H), 7.35(m, 2H), 7.52(m, 2H), 7.58(s, 1H).

Compound 76: m.p. 136-138°C.δ(ppm): 3.72(s, 3H), 3.80(s, 3H), 5.30(s, 2H₂), 6.49(s, 1H), 7.03(m, 2H), 7.20(m, 1H), 7.35(m, 2H), 7.47(m, 1H), 7.57(m, 2H), 7.60(s, 1H).

Compound 77: m.p. 140-142°C.δ(ppm): 2.33(s, 3H), 3.70(s, 3H), 3.78(s, 3H), 5.31(s, 2H), 6.45(s, 1H), 7.14(m, 2H), 7.20(m, 1H), 7.35(m, 2H), 7.50(m, 3H), 7.58(s, 1H).

Compound 78: m.p. 128-130°C.δ(ppm): 3.69(s, 3H), 3.81(s, 3H), 5.36(s, 2H), 6.61(s, 1H), 7.03(m, 2H), 7.20(m, 1H), 7.29(m, 1H), 7.38(m, 2H), 7.60(s, 1H), 7.67(s, 1H), 8.82(m, 1H).

Compound 79: m.p. 126-127°C.δ(ppm): 2.29(s, 3H), 2.33(s, 3H), 3.66(s, 3H), 3.74(s, 3H), 5.23(s, 2H), 6.4 (s, 1H), 6.89(s, 1H), 7.05(m, 2H), 7.17(m, 1H), 7.33(m, 3H), 7.54(s, 1H), 7.70(d, 1H).

Compound 80: m.p. 134-136°C.δ(ppm): 3.68(s, 3H), 3.80(s, 3H), 5.35(s, 2H), 6.58(s, 1H), 7.22(m, 2H), 7.26(m, 1H), 7.37(m, 2H), 7.51(m, 1H), 7.58(s, 1H), 7.75(s, 1H), 8.42(d, 1H).

Compound 81: oil. δ(ppm): 2.29(s, 3H), 2.32(s, 3H), 3.64(s, 3H), 3.71 (s, 3H), 5.25(s, 2H), 6.43(s, 1H), 6.91(m, 2H), 7.13(m, 2H), 7.33(m, 2H), 7.53(s, 1H, CH), 7.72(s, 1H).

Compound 82: m.p. 133-135°C.δ(ppm): 3.66(s, 3H), 3.77(s, 3H), 4.70(d, 2H), 5.26(s, 2H), 6.32(s, 1H), 7.20(m, 3H), 7.35(m, 5H), 7.57(s, 1H).

Compound 83: m.p. 142-144°C.δ(ppm): 2.23(s, 3H), 2.25(s, 3H), 3.70(s, 3H), 3.78(s, 3H), 5.26(s, 2H), 6.44(s, 1H), 7.11(m, 1H), 7.20(m, 2H), 7.35(m, 2H), 7.40(m, 1H), 7.48(s, 1H), 7.53(s, 1H).

Compound 84: thick oil. δ(ppm): 3.63(s, 3H), 3.72(s, 3H), 4.80(s, 2H), 6.37(s, 1H), 6.95(m, 2H), 7.06(m, 2H), 7.40(m, 1H), 7.49(m, 1H), 7.58(s, 1H). 3H), 3.76(s, 3H), 5.25(s, 2H),

Compound 85: m.p. 138-140°C.δ(ppm): 2.31(s, 3H), 3.67(s, 3H), 3.76(s, 3H), 5.25(s, 2H), 6.48(s, 1H), 6.92(d, 1H), 7.20(m, 3H), 7.35(m, 2H), 7.40(m, 1H), 7.55(s, 1H), 7.82(d, 1H).

Compound 86: m.p. 122-124°C.δ(ppm): 3.64(s, 3H), 3.75(s, 3H), 4.60(d, 2H), 5.22(s, 2H), 6.3.2(s, 1H), 7.19(m, 1H), 7.33(m, 7H, 7.42(m, 1H),7.58(s, 1H).

Compound 87: thick oil. δ(ppm): 2.34(s, 3H), 3.70(s, 3H), 3.78(s, 3H), 5.27(s, 2H), 7.19(m, 1H,), 7.24(m, 2H), 7.34(m, 2H), 7.49(m, 1H), 7.55(m, 2H), 7.58(m, 1H).

Compound 88: thick oil. δ(ppm): 3.72(s, 3H), 3.78(s, 3H), 5.31(s, 2H), 6.51(s, 1H), 7.19(m, 1H), 7.29(m, 2H), 7.35(m, 2H), 7.48(m, 1H), 7.57(m, 2H), 7.60(m, 1H).

Compound 89: m.p. 93-95°C.δ(ppm): 1.59(m, 4H), 1.62(m, 2H), 3.68(s, 3H), 3.77(m, 4H), 3.84(s, 3H), 5.27(s, 2H), 6.17(s, 1H), 7.19(m, 1H), 7.34(m, 2H), 7.48(m, 1H), 7.56(s, 1H).

Compound 90: m.p. 142-144°C.δ(ppm): 3.68(s, 3H), 3.75(m, 8H), 3.81(s, 3H), 5.28(s, 2H), 6.27(s, 1H), 7.19(m, 1H), 7.35(m, 2H), 7.46(m, 1H), 7.57(s, 1H).

Compound 91: thick oil. δ(ppm): 1.25(d, 6H), 2.59(m, 2H), 3.60(m, 2H), 3.68(s, 3H), 3.80(s, 3H), 4.53(m, 2H), 5.28(s, 2H), 6.24(s, 1H), 7.19(m, 1H), 7.35(m, 2H), 7.49(m, 1H), 7.56(s, 1H).

### FORMULATION EXAMPLE

### Base on 100% active ingredient(Weight/Weight %)

Example 2: 30% Compound 2 wettable powders

| | |
|---|---|
| Compound 2 | 30% |
| Sodium dodecyl sulfate | 2% |
| Lignin sulfonate | 3% |
| Naphthalene sulfonic acid formaldehyde condensate | 5% |
| Precipitated calcium carbonate | Make up to 100% |

Compound 2 and other components are fully mixed, after smashing through ultrafine pulverizer.

### Example 3: 30% Compound 2 suspension concentrate

| | |
|---|---|
| Compound 2 | 40% |
| Glycol | 10% |
| Nonylphenols polyethylene glycol ether | 6% |
| Lignin sulfonate | 10% |
| Carboxymethyl cellulose | 1% |
| 37% formaldehyde aqueous solution | 0.2% |
| 75% of silicone oil water emulsion | 0.8% |
| Water | make up to 100% |

Fully mixing the compound 2 and other components, suspension concentrate can be obtained, water suspension can be diluted from the concentration of any necessary dilution.

### Example 4: 60% Compound 2 water dispersible granules

| | |
|---|---|
| Compound 2 | 60% |
| Naphthalene sulfonate formaldehyde condensate | 12% |
| Sodium-N-methyl-N-oleyl taurate | 8% |
| Polyvinylpyrrolidone | 2% |
| Carboxymethyl cellulose | 2% |
| Kaolin | make up to 100% |

To mix the compound 2 and other components , after smashing, kneading together with water, added to the granulation 10-100 mesh machine for granulation, then by drying and sieving (at the scope screen).

### BIOLOGICAL TESTING

### Example 5 Determination of insecticidal and acaricidal activity

Determination of insecticidal and acaricidal activity of selected compounds were carried out by following procedure:

Compounds were dissolved in mixed solvent (acetone: methanol=1:1), and diluted to required concentration with water containing 0.1% of Tween 80.

The second instar larvae of armyworm(*Spodoptera exigua),* diamond backmoth (*Plutella xylostella*), green peach aphids (*Myzus persicae*) and mite (*Tetranychus cinnabarinus*) were used in biological test. The method was employed either spraying by airbrush. A test solution (0.5 ml) was sprayed at the pressure of 0.7 kg/cm². Percent mortality was determined after 2-3 days.

Part of test results:
At 600 mg/L, compound 5, showed 100% control of diamond backmoth. Compounds 2, 5, 78, 79, 80 and 83 showed 100% control of mite.
At 150 mg/L, compound 2 showed 100% control of mite.
At 40 mg/L, compounds 78 and 80 showed 100% control of mite. Compound 83 showed more than 95% control of mite.
At 5 mg/L, compounds 2, 78 and 80 showed more than 50% control of mite.
At 10 mg/L, compound 2 showed 100% inhibition acivity on carmine spider mite eggs. At 5 mg/L, compound 2 showed 80% inhibition activity on carmine spider mite eggs..
At 20 mg/L, compound 2 showed 100% control on nymph of carmine spider mite. At 10 mg/L, compound 2 showed 90% control on nymph of carmine spider mite.

### Example 6 Determination of fungicidal activity

Determination of fungicidal activities against plant diseases of selected compounds were carried out by following procedure:

### Determination of fungicidal activity in vitro:

Compounds were dissolved into DMSO to receive three solutions of 2000 mg/L, 667 mg/L and 222 mg/L for test. The solution for inhibition of fungal growth was added in 96 well polystyrene microtiter plates so that all wells receive 1 µl of DMSO solution of test compound, then 79 µl fungus conidial suspension were added into wells to receive the required concentration 25 mg/L, 8.3 mg/L and 2.8 mg/L respectively, which were placed into incubator. After 24 hours incubation, all plates were evaluated for inhibition percent of fungal growth.

### Determination of fungicidal activity in vivo:

Compounds were disolved into acetone to receive stock solution. Deionized water containing 0.1% Tween 80 was added in the previous solution to receive 20 ml test solution. Plants are sprayed by using a turntable sprayer. After 24 hours, plants were innoculated with a conidial suspension. Plants were then transferred into a dew chamber [R.H. (relative humidity) >95%] for infection. After this treatment, plants were removed from the chamber to the greenhouse for normal maintaining, After the infection period, the plants were placed in the greenhouse. After 1 week, the plants were scored for disease control.

### Part of test results in vitro:

At 25 mg/L, compounds, 1, 2, 5 and 12, showed 100% control of rice blast. Compounds 1, 2, 5 and 12 showed 100% control of tomato late blight. Compounds 1, 2, and 12 showed more than 50% control of cucumber grey mold.

At 8.3 mg/L, compounds 1 and 2 showed 100% control of rice blast, Compounds 5 and 12 showed about 80% control of rice blast.

At 2.8 mg/L, compounds 1 and 2 showed 100% control of rice blast, compound 12 showed about 80% control of rice blast.

Part of test results *in vivo:*

At 400 mg/L, compounds 1, 2, 5, and 12 showed 100% control of cucumber downy mildew, compounds 1, 2, 5, and 12 showed 100% control of cucumber anthracnose, compounds 1, 2 and 5 showed more than 80% control of wheat powdery mildew.

At 25 mg/L, compounds 1, 5, and 12 showed 100% control of cucumber downy mildew, compounds 1, 2, 5, and 12 showed 100% control of cucumber anthracnose. Compounds 1, 5 and 12 showed 100% control of corn rust.

At 6.25 mg/L, compound 5, showed more than 80% control of corn rust.

## Claims

1. A substituted pyrimidine ether compound of general formula I: 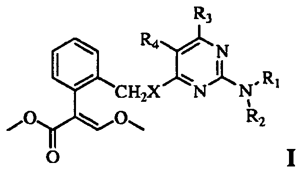 wherein:
R₁ is selected from H or C₁-C₄ alkyl:
R₂ is selected from phenyl, benzyl or pyridyl; each phenyl, benzyl and pyridyl optionally substituted with 1-3 substituents independently selected from the group consisting of halogen, NO₂, CN, C₁-C₃alkyl, C₁-C₃haloalkyl, C₁-C₃alkoxy or C₁-C₃haloalkoxy;
Or NR₁R₂ can join together to form morpholine, piperidine, pyrrolidine or piperazine;
R₃ and R₄ may be the same or different, selected from H, Cl, Br, NO₂, CN, C₁-C₄alkyl, C₁-C₄haloalkyl, C₃-C₆cycloalkyl, C₁-C₃alkoxy or C₁-C₃haloalkoxy;
X is selected from O or S;
Or a salt formed with hydrochloric, phosphoric, acetic, benzenesulfonic or oxalic acid.

2. The compound according to claim 1, wherein:
R₁ is selected from H or methyl;
R₂ is selected from phenyl or pyridyl; each phenyl and pyridyl optionally substituted with 1-3 substituents independently selected from the group consisting of halogen, CN, C₁-C₃alkyl, C₁-C₃haloalkyl, C₁-C₃alkoxy or C₁-C₃haloalkoxy;
Or NR₁R₂ can join together to form morpholine, piperidine, pyrrolidine or piperazine;
R₃ is selected from H, Cl, Br, F, methyl, cyclopropyl or CF₃;
R₄ is selected from H, Cl, Br, F, CH₃ or CN;
X is selected from O ;
Or a said salt.

3. The compound according to the claim 2, wherein:
R₁ is H;
R₂ is selected from phenyl or pyridyl; each phenyl and pyridyl optionally substituted with 1-3 substituents independently selected from the group consisting of Cl, F, CN, methyl, CF₃, OCH₃ or OCF₃ ;
Or NR₁R₂ can join together to form morpholine or piperidine;
R₃ is selected from methyl, cyclopropyl or CF₃;
R₄ is selected from H, Cl or CH₃;
XisO.

4. Use of a compound according to claim 1, as an insecticide, acaricide or fungicide.

5. A composition for use as an insecticide, acaricide or fungicide containing a compound according to claim 1 as an active ingredient, wherein the weight percentage of the active ingredient in the composition is from 0.5-90%.

## Patentansprüche

1. Substituierte Pyrimidinetherverbindung der allgemeinen Formel I: 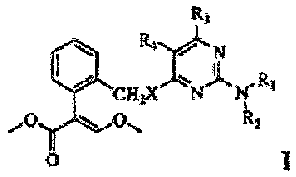 worin:
R₁ aus H und C₁₋₄-Alkyl ausgewählt ist;
R₂ aus Phenyl, Benzyl und Pyridyl ausgewählt ist; wobei jedes Phenyl, Benzyl und Pyridyl gegebenenfalls mit 1 bis 3 Substituenten substituiert ist, die jeweils unabhängig voneinander aus der aus Halogen, NO₂, CN, C₁₋₃-Alkyl, C₁₋₃-Halogenalkyl, C₁₋₃-Alkoxy und C₁₋₃-Halogenalkoxy bestehenden Gruppe ausgewählt sind;
oder NR₁R₂ sich zusammenschließen und Morpholin, Piperidin, Pyrrolidin oder Piperazin bilden kann;
R₃ und R₄ gleich oder verschieden sein können und aus H, Cl, Br, NO₂, CN, C₁₋₄-Alkyl, C₁₋₄-Halogenalkyl, C₃₋₆-Cycloalkyl, C₁₋₃-Alkoxy und C₁₋₃-Halogenalkoxy ausgewählt sind;
X aus O und S ausgewählt ist;
oder ein mit Salz-, Phosphor-, Essig-, Benzolsulfon- oder Oxalsäure gebildetes Salz.

2. Verbindung nach Anspruch 1, worin:
R₁ aus H und Methyl ausgewählt ist;
R₂ aus Phenyl und Pyridyl ausgewählt ist; wobei jedes Phenyl und Pyridyl gegebenenfalls mit 1 bis 3 Substituenten substituiert ist, die jeweils unabhängig voneinander aus der aus Halogen, NO₂, CN, C₁₋₃-Alkyl, C₁₋₃-Halogenalkyl, C₁₋₃-Alkoxy und C₁₋₃-Halogenalkoxy bestehenden Gruppe ausgewählt sind;
oder NR₁R₂ gegebenenfalls zu Morpholin, Piperidin, Pyrrolidin oder Piperazin verbunden ist;
R₃ aus H, Cl, Br, F, Methyl, Cyclopropyl und CF₃ ausgewählt ist;
R₄ aus H, Cl, Br, F, CH₃ und CN ausgewählt ist;
X aus O ausgewählt ist;
oder ein besagtes Salz.

3. Verbindung nach Anspruch 2, worin:
R₁ H ist;
R₂ aus Phenyl und Pyridyl ausgewählt ist; wobei jedes Phenyl und Pyridyl gegebenenfalls mit 1 bis 3 Substituenten substituiert ist, die jeweils unabhängig voneinander aus der aus Cl, F, CN, Methyl, CF₃, OCH₃ und OCF₃ bestehenden Gruppe ausgewählt sind;
oder NR₁R₂ gegebenenfalls zu Morpholin oder Piperidin verbunden ist;
R₃ aus Methyl, Cyclopropyl und CF₃ ausgewählt ist;
R₄ aus H, Cl und CH₃ ausgewählt ist;
X O ist.

4. Verwendung einer Verbindung nach Anspruch 1 als Insektizid, Akarizid oder Fungizid.

5. Zusammensetzung zur Verwendung als Insektizid, Akarizid oder Fungizid, die eine Verbindung nach Anspruch 1 als Wirkbestandteil enthält, worin der Prozentanteil des Wirkbestandteils in der Zusammensetzung 0,5 bis 90 Gew.-% beträgt.

## Revendications

1. Composé d'éther de pyrimidine substitué de formule générale I : 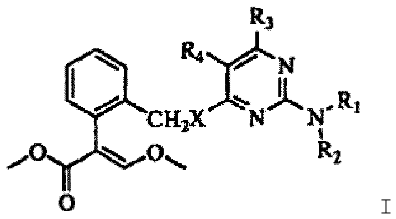 dans lequel :
R₁ est choisi parmi H ou alkyle en C₁-C₄ ;
R₂ est choisi parmi phényle, benzyle ou pyridyle ; chaque phényle, benzyle et pyridyle étant facultativement substitué par 1 à 3 substituants indépendamment choisis dans le groupe constitué d'halogène, NO₂, CN, alkyle en C₁-C₃, halogénoalkyle en C₁-C₃, alcoxy en C₁-C₃ ou halogénoalcoxy en C₁-C₃ ;
ou NR₁R₂ peut être assemblé conjointement pour former morpholine, pipéridine, pyrrolidine ou pipérazine ;
R₃ et R₄ peuvent être identiques ou différents, choisis parmi H, Cl, Br, NO₂, CN, alkyle en C₁-C₄, halogénoalkyle en C₁-C₄, cycloalkyle en C₃-C₆, alcoxy en C₁-C₃ ou halogénoalcoxy en C₁-C₃ ;
X est choisi parmi O ou S ;
ou un sel est formé avec l'acide chlorhydrique, phosphorique, acétique, benzènesulfonique ou oxalique.

2. Composé selon la revendication 1, dans lequel :
R₁ est choisi parmi H ou méthyle ;
R₂ est choisi parmi phényle ou pyridyle ; chaque phényle et pyridyle étant facultativement substitué par 1 à 3 substituants indépendamment choisis dans le groupe constitué d'halogène, CN, alkyle en C₁-C₃, halogénoalkyle en C₁-C₃, alcoxy en C₁-C₃ ou halogénoalcoxy en C₁-C₃ ;
ou NR₁R₂ peut être assemblé conjointement pour former morpholine, pipéridine, pyrrolidine ou pipérazine ;
R₃ est choisi parmi H, Cl, Br, F, méthyle, cyclopropyle ou CF₃ ;
R₄ est choisi parmi H, Cl, Br, F, CH₃ ou CN ;
X est choisi parmi O ;
ou ledit sel.

3. Composé selon la revendication 1, dans lequel :
R₁ est H ;
R₂ est choisi parmi phényle ou pyridyle ; chaque phényle et pyridyle étant facultativement substitué par 1 à 3 substituants indépendamment choisis dans le groupe constitué de Cl, F, CN, méthyle, CF₃, OCH₃, ou OCF₃ ;
ou NR₁R₂ peut être assemblé conjointement pour former morpholine ou pipéridine ;
R₃ est choisi parmi méthyle, cyclopropyle ou CF₃ ;
R₄ est choisi parmi H, Cl ou CH₃ ;
X est O.

4. Utilisation d'un composé selon la revendication 1, en tant qu'insecticide, acaricide ou fongicide.

5. Composition pour utilisation en tant qu'insecticide, acaricide ou fongicide contenant un composé selon la revendication 1 en tant que substance active, dans laquelle le pourcentage en poids de la substance active dans la composition est de 0,5 à 90 %.
